# EUROPEAN PATENT APPLICATION

(11) **EP 1 270 729 A1**
(43) Date of publication of application: **02.01.2003**
(21) Application number: 01909850.8
(22) Date of filing: 10.03.2001
(51) Int. Cl.: C12N 15/56, C12N 9/24, A01H 5/00, A01H 5/08

(54) **DNA MOLECULE CODING FOR A POLYGALACTURONASE OF STRAWBERRY AND THE USES THEREOF**

(30) Priority: 10.03.2000 ES 200000579
(71) Applicant: NEWBIOTECHNIC, S.A., 41092 Sevilla (ES); Universidad de Cordoba, 14013 Cordoba (ES)
(72) Inventor: MUNOS BLANCO, Juan, E-14013 Córdoba (ES); CABALLERO REPULLO, José Luis, E-14013 Córdoba (ES)
(86) International application number: ES0100093
(87) International publication number: WO01066768

(57) **Abstract**

This invention relates to a DNA molecule that codes for a strawberry polygalacturonase (PG) and to its use for modulating the expression of PG in fruits, especially strawberries and for obtaining transgenic plants. The invention also relates to the PG obtained by expression and translation of said DNA molecule and to the use of said enzyme in the degradation of substrates that contain pectins. Of application in Agriculture and the Food Industry.

## Description

### FIELD OF THE INVENTION

This invention relates to a DNA molecule coding for a strawberry polygalacturonase and the use thereof to modulate the expression of polygalacturonase in fruits, especially strawberries. The invention also relates to the polygalacturonase obtained by expression and translation of said molecule of DNA and to the use of said enzyme in the degradation of substrates containing pectins.

### BACKGROUND OF THE INVENTION

The strawberry is a soft fruit consisting of a fleshy receptacle, on the surface of which the achenes (the true fruit) are arranged, joined to the receptacle by means of vascular connections. A characteristic problem of the fruit of the strawberry plant is the length of time it can be kept. The fact that it is a soft fruit, which undergoes a clear softening as it matures and after harvesting makes it a labile and perishable product, both during the collection process and during the storage, transport and distribution processes.

The softening of. the fruit occurs, at least in part, because of the degradation of the middle lamella of the cell wall, the main component of which are the pectin polymers. These consist, in their majority, of chains of α-D-galacturonic acid bound by (1,4) bonds and methoxylated derivatives thereof.

During the ripening of the fruit of the strawberry plant, increases in the activity of pectin methyl esterase (PME) have been observed [Barnes and Patchett, (1976)] (see relevant section in the References), which catalyses the withdrawal of methanol from the pectin converting it into polygalacturonic acid, and cellulase [Archer, (1979); Abeles and Takeda, (1995)], which catalyses the degradation of the cellulose by hydrolysis of the internal β-(1,4) bonds. Similarly, the presence of polygalacturonases (PG), endo-PG and exo-PG have been detected [Hubert, (1984); Nogata et al., (1993); Knee et al., (1977)], although it has been shown that the exo-PG activity reduces as the fruit of the strawberry plant ripens [Nogata et al., (1993)]. The PGs hydrolyse the glycoside bonds of the chain of polygalacturonic acid of the cell walls during the ripening of the fruit. Similarly, it has been observed that the expression of a gene coding for a pectate lyase (PL), which catalyses the cleavage of the (1,4) glycoside bonds that bind the monomers of α-D-galacturonic acid together, increases dramatically with the ripening of the fruit [Medina-Escobar et al., (1997)]. In tomato, corn, potato and carrot, a synergic action of PME, PG and PL has been described [Koch and Nevins, (1989); Wakeley et al., (1998); Zamski and Peretz, (1996)].

The PGs [EC 3.2.1.15] appear to contribute to the softening of the fruit through their action on the pectins of the cell and intercell walls. These enzymes are used in the food industry, mainly in the processing of fruits, greens, vegetables, etc., for example, in the production of wine, fruit juices, etc.

Different genes and complementary DNA sequences have been described (cDNA) which code for the PG of different species of plants, for example, tomato [US patent 4,801,540], peach [Patent application PCT WO 94/05795], or corn [European Patent application EP 651 814].

In some soft fruits, for example, tomato, especially during ripening, PG has been linked to the breakdown of the pectins that constitute the cell wall, a process that accompanies the softening of several types of fruits and which is characteristic of the process of ripening thereof.

A way of delaying the softening of the tomato consists of reducing the enzymatic activity of some of the enzymes implicated in the softening of the fruit, for example, the PG. This can be achieved by reducing the level of expression of the gene coding for said enzymatic activity by means of the use of DNA constructs that contain a part of the gene coding for the enzyme in question in the opposite orientation to normal for its expression [see the US Patent 4,801,540 in which the DNA sequence coding for the PG of tomato is described and its use in the modulation of the expression of plant cells].

The fruit of the strawberry plant is a highly appreciated food product that is widely accepted and of great commercial interest, despite being delicate, labile and perishable.

It would be advantageous to have fruits of the strawberry plant that, while maintaining their organoleptic properties, were to be less labile during the ripening and post-harvest period, in order to increase the time they last both during harvest and during storage, transport and distribution. An alternative way of obtaining this type of fruits of strawberry plants would be to develop some transgenic fruits of strawberry plants that had low or non-existent levels of mRNA corresponding to the PG in order to delay their softening. To do this, it is necessary to identify, isolate and characterise the genomic DNA (gDNA), or complementary DNA (cDNA), which codes for the strawberry PG and/or the mRNA corresponding to said enzyme. However, some authors have reported the absence of endo-PG activity in fruits of strawberry plants, as well as in other fruits such as apples, Japanese parsimmon and melons [McCollum et al., (1989), Ranwala et al., (1992)], although other authors have recently described very low endo-PG activity in such fruits [Lester et al., (1994), Kutsunai et al., (1993), Nogata et al., (1993), Wu et al., (1993), Hadfield et al., (1998)]. This uncertainty along with, if applicable, the low level of enzymatic activity, has made the identification and isolation of the DNA coding for said strawberry enzyme (PG), or of the mRNA corresponding to the transcription of the gene coding for said enzyme, much more difficult, and this may have been why, despite the interest in the problem, it has been impossible to identify, isolate and manipulate said molecules of nucleic acids.

The invention provides a solution to the existing need that comprises the cloning of a strawberry gene, as well as the corresponding cDNA, which codes for a strawberry endo-PG.

Thus, an object of this invention comprises a molecule of DNA coding for a strawberry PG.

An additional object of this invention comprises the use of said molecule of DNA to modulate the expression of the PG in fruits, preferably in fruits of strawberry plants.

Another additional object of this invention comprises a DNA construct that comprises all or part of said molecule of DNA, as well as a vector containing said DNA molecule or construct and a cell transformed with said vector.

Another additional object of this invention comprises the use of said DNA molecule, or said DNA construct, in the contruction of transgenic plants that express a modulated PG enzymatic activity, for example, transgenic plants that possess levels of mRNA corresponding to highly reduced or non-existent PGs. The resulting transgenic plants constitute another additional object of this invention.

Another additional object of this invention comprises a protein with polygalacturonase activity obtained by expression and translation of said DNA molecule.

Another additional object of this invention comprises the use of said protein with polygalacturonase activity in the degradation of substrates susceptible to the action of said enzymatic activity.

Another additional object of this invention comprises an enzymatic preparation comprising said protein with polygalacturonase activity.

### BRIEF DESCRIPTION OF THE FIGURES

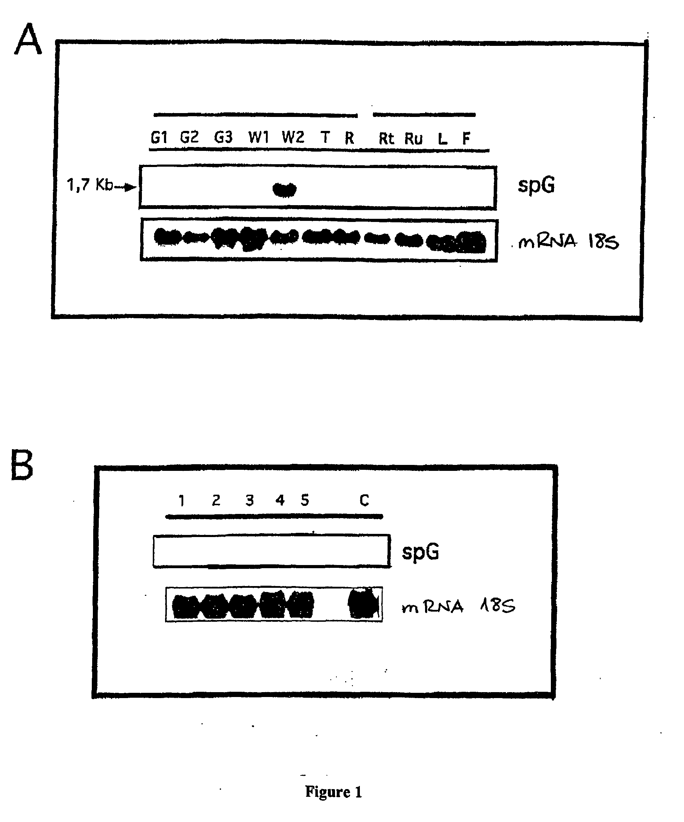

Figure 1 illustrates the model of expression of the space-time development of the *spG* gene in strawberry (Figure 1A) and the effects of withdrawal of the achenes and of the treatment with auxins on the expression of the *spG* gene (Figure 1B). Figure 1A shows the result of a Northern blot analysis of 20 pg of total RNA isolated from samples collected from the G1, G2, G3 and W1 stages (stages in the growth of the fruit of the strawberry plant), W2, T and R stages (stages in the ripening of the fruit of the strawberry plant) and the roots (Rt), leaves (L), flowers (F) and stolons (Ru). The size of the hybridisation transcript was 1.7 Kb. The hybridisation and astringency conditions are described in Example 2.2 [Isolation of RNA and Northern blot analysis]. Figure 1B shows the result of a Northern blot analysis with RNA isolated from fruits of the strawberry plant at the G2 stage after withdrawal of achenes, at different times: (1) 0 h; (2) 24 h; (3) 48 h; (4) 72 h; (5) 96 h; and of fruits of the strawberry plant at the G2 stage without achenes (96 h) treated with NAA auxin (C).

### DETAILED DESCRIPTION OF THE INVENTION

The invention provides a DNA molecule coding for a strawberry PG, hereinafter the DNA molecule of the invention, selected from:
a) a DNA sequence that comprises the sequence of nucleotides identified as SEQ. ID. No.: 1;
b) a sequence of DNA that comprises the sequence of nucleotides identified as SEQ. ID. No.: 5; and
c) a sequence of DNA which is analogous to the sequence defined in a) or in b) that
   i) is substantially homologous with the DNA sequence defined in a) or in b); and/or
   ii) codes for a polypeptide that is substantially homologous to the protein coded for by the DNA sequence defined in a) or in b).

In the sense used in this description, the term "analogous" includes any sequence of DNA coding for an enzyme that possesses, at least, PG activity that has properties i)-ii) mentioned above. Typically, the analogous DNA sequence:
- can be isolated from another species that produces a PG on the basis of the sequence of nucleotides shown in SEQ. ID. No.: 1, or in SEQ. ID. No.: 5, or
- is constructed on the basis of the sequence of nucleotides shown in SEQ. ID. No.: 1 or in SEQ. ID. No.: 5, for example, through the introduction of substitutions of conserving nucleotides, in other words, nucleotides that do not give rise to another sequence of amino acids of the PG coded for by said sequence of nucleotides shown in SEQ. ID. No.: 1 or in SEQ. ID. No.: 5, but which correspond to the use of codons of the host organism for the production of the protein, or else through the introduction of replacements of nucleotides that give rise to a different sequence of amino acids and, therefore, possibly, to a different protein structure that might give rise to a mutant protein with properties different to those of the native protein. Other examples of possible modifications include the insertion of one or more nucleotides into the sequence, the addition of one or more nucleotides at either end of the sequence, or the deletion of one or more nucleotides at either end or in the interior of the sequence. For example, the analogous DNA sequence can be a sub-sequence of the sequence of nucleotides shown in SEQ. ID. No.: 1 or in SEQ. ID. No.: 5.

In general, the analogous DNA sequence is substantially homologous to the sequence of nucleotides identified as SEQ. ID. No.: 1 or SEQ. ID. No.: 5. In the sense used in this description, the expression "substantially homologous", applied to the sequences of nucleotides, means that the sequences of nucleotides in question has a degree of identity, at the nucleotide level, of at least 70%, preferably of, at least 85%, or more preferably, at least 95%.

The DNA molecule of the invention may come from any variety of strawberry, for example from the strawberry (*Fragaria x ananassa*) or from a host organism transformed with said DNA molecule.

Alternatively, the DNA molecule of the invention can be isolated by means of conventional techniques from the DNA of any other species by means of the use of probes or of oligonucleotides prepared from the information on the DNA sequence provided in this specification.

In a particular embodiment, the DNA molecule of the invention is a cDNA molecule of the mRNA corresponding to the gene of the PG of strawberry, in particular, of an endo-PG of strawberry (*Fragaria x ananassa*), whose sequence of nucleotides comprises the sequence of nucleotides shown in SEQ. ID. No.: 1. SEQ. ID. No.: 1 corresponds to the sequence of a partial fragment of cDNA of the mRNA of the PG of strawberry.

In another particular embodiment, the DNA molecule of the invention is a genomic DNA molecule (gDNA) corresponding to the *spG* gene coding for the PG of strawberry, related to the specific ripening of the fruit (*Fragaria x ananassa*), which has been characterised on a molecular level and physiologically and whose sequence of nucleotides comprises the sequence of nucleotides shown in SEQ. ID. No.: 5. The comparative analysis of the sequence deduced from the protein has shown that this gene corresponds to a gene coding for an endo-PG Clade A.

The DNA molecule of the invention can be obtained by using conventional methods known to those skilled in the art, for example, by means of a process that comprises the extraction of the mRNA corresponding to the transcription of the gene coding for the PG from a producing organism of said enzyme, obtaining a first chain of cDNA by reverse transcription of the corresponding mRNA, the synthesis of a second chain of cDNA, complementary to the first one, to obtain a double-chain cDNA, the joining of some linkers for its insertion into plasmids or phages for its propagation in, for example, a bacterial system and the identification of the clones that carry the desired cDNA.

In a particular embodiment [see Example 1], a partial clone of cDNA corresponding to mRNA of the PG of strawberry fruit is obtained by means of a process that comprises applying the DDRT-PCR technique [Differential Display Retro-transcriptase-PCR] to mRNA from unripe fruits of the strawberry plant that are expressed differentially in unripe strawberry fruits which had had their achenes removed but which was not done (or was done at virtually undetectable levels) in strawberry fruits in the same stage of ripening but which contained the achenes. For this, the mRNA of said fruits was extracted, submitted to a reverse transcription reaction [RT] to obtain the corresponding single chain cDNA and the cDNA that presented a differential expression [in other words, those which were expressed in the strawberry fruits without achenes but which was not done (or was done at virtually undetectable levels) in the strawberry fruits with achenes] were extracted and amplified by PCR. The products of the amplification [which correspond to the mRNA expressed in the unripe fruits without achenes but not in the fruits with achenes, and which contained the sequence of nucleotides coding for the strawberry PG] were subcloned into appropriate vectors which were used to transform bacteria. Then, the DNA corresponding to the recombinant plasmid was extracted, purified and sequenced. The partial sequence obtained [SEQ. ID. No.: 1] was compared with the sequences deposited in the data bases using the BLAST program of the National Center for Biotechnology Information (NCBI, United States). Comparison of the sequences showed that the DNA sequence obtained coding for the strawberry PG showed a relatively high homology of sequence only and exclusively with other sequences of higher plants that code for endo-PG.

In Example 2, the cloning of the *spG* gene coding for a strawberry PG provided by this invention is described. The cloning process comprises the isolation of cDNA corresponding to the mRNA of the strawberry fruit PG, as was mentioned earlier. Using this cDNA as a probe, a gene (*spG*) was isolated that was completely sequenced [SEQ. ID. No.: 5]. Comparison between the sequence of the spG gene and the sequences present in the database showed a significant sequence identity at the level of amino acids only with sequences corresponding to endo-PGs of higher plants.

The invention provides, in addition, a DNA construct, hereinafter, the DNA construct of the invention, that comprises the entire molecule of DNA of the invention or a fragment of, at least, 8 consecutive nucleotides of the DNA molecule of the invention and a functional transcription initiation region in plants. In said construct, either terminal (3' or 5') of the entire molecule or the fragment of the DNA molecule of the invention may be bound to the 3' terminal of said transcription initiation region. The DNA construct of the invention can also contain, operatively bound, a transcription termination sequence. In a particular embodiment, said transcription initiation region is functional in strawberry plants.

The DNA molecule of the invention, or the DNA construct of the invention, may be inserted into the appropriate vector. Therefore, the invention also relates to a vector, such as an expression vector, that comprises said molecule of DNA, or a construct containing it. The choice of vector will depend on the host cell in which it is to subsequently to be introduced. By way of example, the vector where said DNA sequence is introduced may be a plasmid or a vector that, when it is introduced into a host cell, is integrated into the genome of said cell and is replicated along with the chromosome (or chromosomes) in which it has been integrated.

In the vector provided by this invention, the DNA molecule of the invention will be connected operatively to a promoter and to a termination sequence. The promoter may be any DNA sequence that shows transcriptional activity in the chosen host cell and may derive either from genes that code for homologous proteins or heterologous proteins of the host cell. The processes used to bind the DNA sequence of the invention to the promoter and to the termination sequence, respectively, and to insert said construct into a vector are well known to those skilled in the art and have been described, for example, by Sambrok et al., (1989).

The invention also provides a cell that comprises a DNA sequence of the invention, or a DNA construct that contains said sequence or said vector mentioned above. The host cells that can be transformed with the DNA sequence of the invention can be prokaryotic cells or, preferably, eukaryotic, such as cells of vegetable tissue. The transformation of cells of vegetable tissues can also be performed by conventional methods. For a review of genetic transfer to plants, including vectors, methods for DNA transfer, etc., see for example the book entitled "Ingenieria genética y transferencia génica", by Marta Izquierdo, Pub. Pirámide (1999), in particular, chapter 9, entitled "Transferencia génica a plantas", pages 283-316.

The invention also provides a protein with polygalacturonase activity, hereinafter PG of the invention, that has a sequence of amino acids selected from:
a) a sequence of amino acids that comprises the sequence of amino acids shown in SEQ. ID. No.: 2,
b) the sequence of amino acids deduced from the sequence of nucleotides shown in SEQ. ID. No.: 5, and
c) a sequence of amino acids substantially homologous and functionally equivalent to the sequences of amino acids defined in a) or in b).

In the sense used in this specification, the expression "substantially homologous" means that the sequences of amino acids in question have a degree of identity, at the amino acid level of, at least, 70%, preferably of at least 85%, and more preferably of at least 95%.

Similarly, in the sense used in this specification, the expression "functionally equivalent" means that the protein in question has polygalacturonase activity.

In a particular embodiment, the PG of the invention is a strawberry endo-PG (*Fragaria x ananassa*) which has a sequence of amino acids that comprises the sequence of amino acids shown in SEQ. ID. No.: 2. SEQ. ID. No.: 2 corresponds to the sequence of amino acids of a fragment of a strawberry PG and has been deduced from the sequence of nucleotides of the partial fragment of the cDNA of the mRNA corresponding to the gene of the strawberry PG shown in SEQ. ID. No.: 1.

In another particular embodiment, the PG of the invention is a strawberry endo-PG (*Fragaria x ananassa*) which has the sequence of amino acids deduced from the sequence of nucleotides shown in SEQ. ID. No.: 5, identified at times in this specification as sPG or strawberry endo-PG. The comparison between SEQ. ID. No.: 5 and the sequences present in the databases showed a sequence identity at an amino acid level significant only with sequences corresponding to endo-PGs of higher plants. The computer analysis of the ORF of the *spG* gene showed a protein derived from 401 amino acids. Just as in all the endo-PGs of higher plants, the protein contains a hydrophobic peptide signal at the amino terminal, with a potential cleavage site between residues 22 and 23, the cleavage of which gives rise to a mature protein of 40.69 KDa, with a pI of 7.91. Potential sites of N-glycosylation (N-X-S/T) have been found at the 212 and 333 positions of the amino acids. In accordance with the results obtained in relation to the sequence of amino acids, said sPG protein can be classified within the group of PG Clade A proteins. Analysis of the alignments of all the eukaryotic and prokaryotic sequences showed the existence of 4 conserved domains which are supposed to be implicated in the PG activity (I to IV). In addition, a highly conserved His residue is also present (GHG motifs, in domain III; residue 245) to which a catalytic function has been assigned in all the PGs sequenced until present. Similarly, a Tyr residue is also found in the sPG protein at position 315 which is strictly conserved in the PGs, and which has also been shown to be essential for PG activity. In the sPG protein, the residues of conserved cysteines are also observed among the higher plant species and fungi. The space-time relationships and the hormonal model of genetic expression appear to suggest a relationship between the expression of the gene coding for the sPG and the process of ripening of the strawberry fruit. The results suggest a probable role of this enzyme in the release of oligosaccharines from the cell wall [see Example 2].

The PG of the invention can be obtained by means of a method that comprises culturing a suitable host cell that contains the DNA molecule of the invention, or a DNA construct of the invention, under conditions that allow the production of the protein and its recovery from the culture medium.

The PG of the invention can be alternatively obtained from a producing organism thereof by means of a process that comprises the culture of the producing organism, for example, unripe fruits of strawberry without achenes, under appropriate conditions for the expression of said enzyme, and, subsequently, recovering said enzyme.

The invention also provides an enzymatic preparation comprising the PG of the invention, optionally along with one or more proteins which may have different enzymatic activities, for example, different enzymatic activities required for the modification or degradation of microbial cell walls. By way of example, said enzymatic preparation may include lytic, cellulolytic, mananolytic, chitinolytic or proteolytic enzymes, such as cellulases, pectate lyases, pectin methyl esterases, pectin lyases, glucanases, mananases, chitinases, chitosanases, proteases, manosidases, etc.

The enzymatic preparation of the invention, which may contain between 0.01% and 100% by weight of the PG of the invention, may be prepared by conventional methods and may be presented in liquid or solid form, for example, in the form of a granulate. The enzymatic preparation may contain additives, for example, stabilisers that prolong the stability thereof.

The PG of the invention, as well as the enzymatic preparation provided by this invention, may be used for degrading substrates that contain pectins, products essentially comprised by residues of α-D-galacturonic acid bound by (1,4) glycoside bonds, for example, cell walls and intracellular regions of plants, as well as cell walls of some algae. Although it is not intended to be tied to any particular theory, it seems that the degrading action of the proteins of the PG of the invention is carried out by means of a mechanism comprising the hydrolysis of the (1,4) glycoside bonds present in the chain of polygalacturonic acid contained in the pectins.

The PG of the invention is of importance in the food industry, in particular, in the industry of processing soft fruits, such as strawberries, raspberries, blackberries, cranberries, grapes, tomatoes, etc., for example, for obtaining jams, juices, yogurts, etc. The use of the PG of the invention in the food industry provides an added value to said food products given that it could accelerate and/or make cheaper their processing, increase their production, and change the texture of the food products themselves, generating a greater diversity of products and even the appearance of new products on the market.

The DNA molecule of the invention can be used in processes for improving soft fruit, for modulating the expression of PG in fruit, altering the ripening of the fruit and delaying the process of softening of the fruit. In a particular embodiment, the DNA molecule of the invention is used to obtain transgenic plants that possess levels of mRNA corresponding to PG very much reduced or non-existent. In order to obtain these transgenic plants, conventional antisense mRNA techniques can be used and/or overexpression (silencing by sense), or others, for example, using binary vectors or other vectors available for the different techniques of transformation of plants that are currently used.

Therefore, the invention also relates to a transgenic cell of a plant that comprises a DNA construct of the invention that has a promoter, functional in said plant, operatively bound to a sub-sequence of DNA of at least 8 nucleotides, derived from a DNA molecule of the invention, said DNA sub-sequence being bound to the promoter in an opposite orientation to that of its expression.

A transgenic plant that comprises, at least, one of said transgenic cells, constitutes an additional object of this invention. In a particular embodiment, said transgenic plant is a strawberry plant. In other particular embodiments, the transgenic plants are plants that produce soft fruits, such as raspberries, blackberries, cranberries, grapes, tomatoes, etc.

The invention also relates to a method for delaying the softening of soft fruits or for altering the ripening of soft fruits, which comprises introducing into a soft-fruit producing plant a DNA construct that has a promoter, functional in said plant, operatively bound to a sub-sequence of DNA of at least 8 nucleotides, derived from the DNA molecule of the invention, said sub-sequence of DNA being in an opposite orientation to that of its expression. In a particular embodiment, the invention provides a method for delaying the softening of strawberry fruits, or for altering the ripening of soft fruits, which comprises introducing into a soft-fruit producing plant a DNA construct that has a promoter, functional in said plant, operatively bound to a sub-sequence of DNA of at least 8 nucleotides, derived from the DNA molecule of the invention, said sub-sequence of DNA being in an opposite orientation to that of its expression.

In another embodiment, the invention also relates to a method for altering the ripening of soft fruits that comprises introducing into a soft-fruit producing plant a DNA construct that has a promoter, functional in said plant, operatively bound to a sub-sequence of DNA of at least 8 nucleotides, derived from the DNA molecule of the invention, said subsequence of DNA being bound to the promoter in the opposite orientation to that of its expression. In a particular embodiment, the invention provides a method for altering the ripening of strawberry fruits that comprises introducing into a strawberry plant a DNA construct that has a functional promoter in said plant, operatively bound to sub-sequence of DNA of at least 8 nucleotides, derived from the DNA molecule of the invention, said subsequence of DNA being bound to the promoter in the opposite orientation to that of its expression.

The use of the DNA molecule of the invention, in particular, of a cDNA molecule coding for a strawberry PG, of whole or partial length, by means of any type of technique, may generate transgenic plants that yield fruits with better qualities of hardness, and/or with an altered ripening pattern and, as a result, less labile strawberry fruits that are more resistant and lasting, without losing its organoleptic qualities, with the consequent advantage in their gathering, storage, transport and marketing, in general, of transgenic fruits, which provides them with added economic value.

The following examples serve to illustrate the present invention and should not be considered as limiting the scope thereof.

### EXAMPLE 1

### Obtaining a partial clone of cDNA of a strawberry PG

In order to obtain a partial clone of cDNA corresponding to mRNA of a strawberry PG, a strategy was followed comprising the use of the DDRT-PCR technique, with amplification using PCR the cDNA fragments obtained from the mRNA that were expressed in unripe strawberry fruits from which the achenes had been withdrawn but not in strawberry fruits of the same stage of ripening from which the achenes had been withdrawn. The products of amplification were subcloned into vectors and with said vectors, the cells of *E. coli* were transformed, selecting the transforming cells that contained the vector with the cDNA insert corresponding to the strawberry PG, which was isolated, purified and sequenced. The sequence obtained was compared with other sequences of DNA that code for endo-PG of other higher plants.

### 1.1 Extraction of mRNA

In order to obtain mRNA corresponding to the transcription of the gene coding for strawberry PG the total RNA both from strawberry fruits in growth stage G2 (green unripe fruits of an intermediate size) and G2 fruits whose achenes had been removed was extracted and they were left joined to the parent plant for 6 days. For the extraction of total RNA both from the fruits with and without achenes, the method described by Manning (1991) was followed.

### 1.2 DDRT-PCR

Once the total RNA had been extracted from both types of fruit, the populations of mRNA of each fruit were compared among themselves using the DDRT-PCR technique. For this, the commercial kit from the company GENOMYX was used (Beckman Instruments Inc.) "Hieroglyph mRNA Profile Kit System for Differential Display Analysis".

The conditions of the DDRT-PCR which include: synthesis of the anchored subpopulations of single chain cDNA; amplification thereof by PCR, using arbitrary indicators labelled with ³²P; separation and comparison of the resulting populations of double strand cDNA by means of polyacrylamide gel electrophoresis; recovery, from the gel, of the differentially expressed cDNA fragments; and amplification thereof; were those that are indicated in the user manual of the aforementioned kit "Hieroglyph mRNA Profile Kit System for Differential Display Analysis".

Once the cDNA fragments had been amplified by PCR, they were purified from the PCR mixture using the kit "Concert^{TM} rapid PCR purification system" from the company LIFE TECHNOLOGIES (Gibco BRL Products), following the method described in the instruction manual.

The cDNA that presented a differential expression in the strawberry fruits without achenes compared to the strawberry fruits with achenes were extracted and amplified by PCR. Both for the reverse transcription reaction (RT) and for PCR reactions, the anchored oligonucleotide initiator AP1 [SEQ. ID No.: 3] and the arbitrary oligonucleotide initiator ARP3 [SEQ. ID. No.: 4] were used.

After this, the products of the amplification, which corresponded to the mRNA expressed in the fruits without achenes but not in fruits with achenes, and which contained the nucleotide sequence that coded for the strawberry PG, were subcloned into a pGEM-Teasy vector from the company PROMEGA CORPORATION (Madison, United States) following the instructions of the manufacturer.

The fragments of cDNA contained in the recombinant plasmid were sequenced in an automatic sequencer ABI 310 using the kit Taq DyeDeoxy Terminator Cycle Sequencing from APPLIED BIOSYSTEMS (California, United States), obtaining the nucleotide sequence shown in SEQ. ID. No.: 1 (which corresponds to a part of the DNA sequence coding for a strawberry PG).

Next, the cells of *E. coli* were transformed with said vector, checking that the transforming cells contained the vector with the cDNA insert corresponding to the strawberry PG, by means of conventional techniques described by Sambrok et al., (1989). Next, the DNA corresponding to the recombinant plasmid was extracted, purified and sequenced using an automatic DNA sequencer and conventional techniques described by Sambrok et al., (1989). The sequence obtained [SEQ. ID. No.: 1] was compared with the sequences deposited in the databases using the BLAST program from the National Center for Biotechnology Information (NCBI, United States). The comparison of the sequences showed that the DNA sequence obtained coding for strawberry PG showed a relative high sequence homology only and exclusively with other sequences of higher plants that code for endo-PG.

### EXAMPLE 2

### Cloning of the spG gene

The *spG* gene coding for a strawberry PG related to the specific ripening of the fruit (*Fragaria x ananassa* c.v. Chandler) has been cloned and characterised at a molecular level and physiologically. The comparative analysis of the sequence derived from the protein has shown that this gene corresponds to a gene coding for an endo-PG Clade A. In addition, the space-time relationship and the hormonal genetic expression model seem to suggest a relationship between the expression of this gene and the process of ripening of the strawberry fruit. The results suggest a probable role of this enzyme in the release of oligosaccharines from the cell wall.

### MATERIALS AND METHODS

### 2.1 Isolation of cDNA from the spG gene of strawberry

The cDNA corresponding to the *spG* gene of strawberry was isolated using the technique of DDRT-PCR comparing 2 different populations of RNA in strawberry fruit corresponding to green unripe fruit with achenes and green unripe fruits without achenes. The assay for selecting the true positives was performed using reverse Northern blots. The positive cDNA fragments were then cloned into the pGEM-T Easy vector (PROMEGA) following the instructions of the manufacturer and then fully sequenced.

### 2.2 Isolation of the RNA and Northern blot analysis

The total RNA from the fruit of the stages indicated and from roots, leaves, flowers and stolons was isolated following the method described by Manning, (1991). The Northern blot analysis and the treatment with auxins followed was as described by Medina-Escobar et al., (1997). In order to form hybrids with RNA, cDNA from *spG* was used as a probe. Similarly, cDNA corresponding to the 18S fraction of the ribosomal RNA was used as a control for equal loading of the RNA samples.

### 2.3 Cloning of the genomic DNA of the spG gene

A gene library of *Fragaria x ananassa* cv. Chandler in λ-FIX was searched using conventional methods. The cDNA corresponding to *spG* labelled with ³²P was used as a probe to search approximately 400,000 recombinant phages.

### 2.4 Isolation of DNA and Southern Blot analysis

The genomic DNA was extracted from strawberry from the achenes removed from the strawberry fruits in the W1 stage as described by Medina-Escobar et al., (1997). The DNA (5 µg) was digested with restriction enzymes and a Southern blot analysis was performed according to the method described by Medina-Escobar et al., (1997).

### RESULTS AND DISCUSSION

By comparison, using the DDRT-PCR technique, of the populations of mRNA corresponding to the genes expressed in the green unripe stage (G2) of the strawberry fruit with those expressed in green unripe fruit with no achenes, partial cDNA was isolated corresponding to an mRNA coding for an endo-PG [ADNc-*spG*] (see Example 1). Using this cDNA as a probe, a gene was isolated (*spG*) which was sequenced in its entirety [SEQ. ID. No.: 5]. Comparison between the sequence of the *spG* gene and the sequences present in the databases showed a significant sequence identity at the amino acid level only with sequences corresponding to endo-PGs of higher plants. The computer analysis of the ORF of the *spG* gene showed a protein derived from 401 amino acids. Just as in all the endo-PGs of higher plants, the protein contains a hydrophobic peptide signal at the amino terminal with a potential cleavage site between residues 22 and 23. The cleavage of the peptide signal gives as a result a mature protein of 40.69 KDa, with a pI of 7.91. Potential sites of N-glycosylation (N-X-S/T) have been found at the positons of amino acids 212 and 333. In accordance with the results obtained regarding the sequence of amino acids, said sPG protein can be classified within the group of proteins PG Clade A, a group that includes PGs related to the processes of ripening and abscission of the fruit [Haldfield & Bennett, (1998)]. The analysis of the alignments of all the sequences of eukaryotic and prokaryotic PGs showed the existence of 4 conserved domains which are supposed to be implicated in the PG activity (I to IV). In addition, a highly conserved residue of His (GHG motifs, on domain III; residue 245) is also present in the endo-PG of strawberry provided by this invention (sPG). This residue has been assigned a catalytic function in all the PGs that have been sequenced up until present. Similarly, a residue of Tyr is also found on the sPG protein at the 315 position which is strictly conserved in the PGs, and which has also been shown to be essential for the PG activity. In addition, in the sPG protein, the residues of cysteine conserved between species of higher plants and fungi were also observed. The hybridisation pattern observed for the *spG* gene by means of Southern Blot analysis suggests the presence of a small multigenic PG family on the strawberry genome. Alternatively, this could also be due to the level of ploidy in the strawberry.

The time-space and hormonal model expression for the *spG* gene has been studied. As can be seen in Figure 1A, a model of expression specific to the fruit was observed. Furthermore, the *spG* gene was expressed exclusively in two states of ripening of different fruits (W1 and W2), observing greatest levels of transcription in the W2 stage (Figure 1A). However, expression of the gene in the growth stages (G1, G2 and G3) or in the last stages of ripening (T and R) was not found. Said model of genetic expression seems to support an intimate relationship between the physiological role of the sPG protein and the process of ripening of the fruit. During the ripening of the fruit, it is clearly established that the degradation of the cell wall leads to the softening of the fruit [Perkins-Veazie, (1995)]. At the present moment, and in a similar way to that found in other fruits, some hydrolytic enzymes of the cell wall such as pectin methyl esterase, pectate lyase and cellulase, have been implicated in the softening of the strawberry fruit [Perkins-Veazie, (1995); Medina-Escobar et al., (1997); Trainotti et al., (1999)]. In addition, a strong induction in the expression of genes that code for enzymes that degrade the cell wall have been found, such as cellulase and pectate lyase, during all stages of ripening and softening of the strawberry fruit. Therefore, the model of expression found for the spG gene is rather complicated and does not appear to support a clear relationship between its expression and the softening of the fruit. In accordance with this, a high level of transcription was found only in the first stages of ripening of the fruit (stage W2), where there is no evident softening of the strawberry fruit. Healthy fruit, the areas of abscission and the fungal and bacterial phytopathogens all produce endo-PG activities. These enzymes act on the pectins *in vivo* to generate biologically active oligosaccharines (oligo-GalAs) [Dumville & Fry, (2000)]. In addition, in fruits with healthy ripening, there is strong evidence to support the production of oligogalacturonides *in vivo.* The application of the oligo-GalAs to the growing tissues has an effect on different processes [Dumville & Fry, (2000)]. It has also been shown that the external application of pectic oligosaccharines induces the ripening of the fruit of tomato plants and the fruits of citric plants [(Dumville & Fry, (2000)]. In this sense, the model of space-time expression found for the *spG* gene could be consistent with a hypothetical role for this endo-PG in the production of oligogalacturonide oligosaccharines. The sudden and narrow peak of expression right at the start of the process of ripening of the fruit appears to support this hypothesis. These molecules should be potentially implicated in the regulation of some metabolic processes related to the ripening of the strawberry fruit as has been proposed for tomatoes [Dumville & Fry, (2000)]. Alternatively, the sPG protein could be implicated in the hydrolysis of some very specific bonds of the pectin network of the cell wall which could be necessary for subsequent and generalised degradation of the pectins of the cell wall by other enzymes able to degrade the cell wall, for example, pectate lyases.

It has been previously demonstrated that, in the strawberry fruit, the expression of some genes related to the ripening are directly or indirectly regulated by the auxins produced in the achenes. The auxins stimulate the expression of the receptacle but inhibit its ripening [Perkin-Veazie, (1995); Medina-Escobar et al., (1997)]. Therefore, to determine whether the *spG* gene of strawberry is also under hormonal control, some Northern Blot tests were performed with RNA isolated from fruits with achenes and fruits without achenes treated with or without the auxin NAA. As is shown in Figure 1B, a clear increase in the level of transcription of the *spG* gene was found in strawberry fruits without achenes in the G2 stage after 5 days. However, expression of the *spG* gene in fruits with achenes was not detected in the fruits with achenens, which indicated a direct or indirect repression of this gene by the achenes on elongating the fruit. In addition, these increases in the levels of transcription of *spG* reverted in fruits without achenes in the G2 stage treated with NAA, which appears to suggest that the expression of the *spG* gene is regulated by auxins. Similar results have been obtained for other genes related to the ripening involved in the disassembly of the cell wall, such as pectate lyase and cellulase [Medina-Escobar et al., (1997); Trainotti et al., (1998)], and also with those related to other metabolic processes related to ripening [Manning, (1998)]. In this sense, diverse evidence seems to suggest that the pectic. oligosaccharines antagonise the action of the auxin when they are applied at very low levels in physiological processes such as growth induced by auxin of segments of the stalk of peas, the rooting stimulated by auxins in explants of leaves and in the auxin dependent somatic embryogenesis [Creelman & Mullet, (1997)]. Similar results were found with the gene coding for a PG involved in processes of abscission in which treatment with IAA inhibited the genetic expression in the area of abscision [Kalaitzis et al., (1995)].

### REFERENCES

Abeles F.B., and Takeda F., (1990). Cellulase activity and ethylene in ripening strawberry and apple fruits. Sci. Hort., 42:269-275.

Archer S.A., (1979). Pectolytic enzymes and degradation of pectin associated with breakdown of sulphited strawberries. J. Sci. Food Agric., 30:692-703.

Barnes M.F., and Patchet B.J., (1976). Cell degrading enzymes and the softening of senescence strawberry fruit. J. Food Sci., 41:1392-1395.

Creelman R.A. & Mullet J.E., (1997). Oligosaccharins, brassinolides, and jasmonates: nontraditional regulators of plant growth, development and gene expression. The Plant Cell, 9, 1211-1233.

Dumville J.C., & Fry S.C., (2000). Uronic acid-containing oligosaccharins: Their biosynthesis. Degradation and signalling role in non-diseased plant tissues. Plant Physiology and Biochemistry, 38, 125-140.

Haldfield K.A. & Bennett A.B., (1998). Polygalacturonases: many genes in search of a function. Plant Physiology, 117, 337-343.

Hadfield K.A., Rose J.K.C., Yaver D.S., Berka R.M., and Bennett A.B., (1998). Polygalacturonase gene expression in ripe melon fruit supports, a role for polygalacturonase in ripening associated pectin disassembly. Plant Physiol. 117:363-373.

Hubert DJ, (1984). Strawberry fruit softening the potential roles of polyuronides and hemicelluloses. J. Food Sci., 49: 1310-1315.

Kalaitzis P., Koeheler S.M., Tucker M.L., (1995). Cloning of a tomato polygalacturonase expressed in abcission. Plant Molecular Biology, 28, 647-656.

Knee M., Sargent J.A., and Osborne D.J., (1977). Cell wall metabolism in developing strawberry fruits. J. Exp. Bot. 28:377-396.

Koch and Nevins, (1989). Tomato fruit cell wall. I. Use of purified tomato polygalacturonase and pectinmethylesterase to identify developmental changes in pectins. Plant Physiology, 91:816-822.

Kutsunai S.Y., Lin A.C., Percival F.W., Laties G.G., and Christoffersen R.E., (1993). Ripening-related polygalacturonase cDNA from avocado. Plant Physiol. 103:289-290.

Lester D.R., Speirs J., Orr G., Brady C.J., (1994). Peach (*Prunus persica*) endopolygalacturonase cDNA isolation and mRNA analysis in melting and nonmelting peach cultivars. Plant Physiol. 105:225-231.

Manning K., (1991). Isolation of nucleic acids from plants by differential solvent precipitation. Anal. Biochem. 195:45-50.

Manning K., (1998). Isolation of a set of ripening-related genes from strawberry: their identification and possible relationship to fruit quality traits. Planta, 205, 622-631.

McCollum T.G., Huber D.J., and Cantliffe D.J., (1989). Modification of polyuronides and hemicelluloses during muskmelon fruit softening. Physiol. Plant 76:303-308.

Medina-Escobar N., Cardenas J., Moyano E., Caballero J.L., Mufioz-Blanco J., (1997). Cloning, molecular characterization and expression pattern of a strawberry ripening-specific cDNA with sequence homology to pectate lyase from higher plants. Plant Mol. Biol. 34:867-877.

Nogata Y., Ohta H., and Voragen A.G.J., (1993). Polygalacturonase in strawberry fruit. Phytochemistry, 34: 617-620.

Perkins-Veazie P., (1995). Growth and ripening of strawberry fruit. Horticultural Review, 17, 267-197.

Ranwala A.P., Suematsu C., and Masuda H., (1992). The role of β-galactosidases in the modification of cell wall components during muskmelon fruit ripening. Plant Physiol. 100:1318-1325.

Sambrok et al., (1989). Molecular Cloning. A Laboratory Manual, Cold Spring Harbor, NY, (1989).

Trainotti L., Spolaore S., Pavanello A., Baldan B., Casadoro G., (1999). A novel E type endo-β-1,4-glucanase with a putative cellulose-binding domain is highly expressed in ripening strawberry fruits. Plant Molecular Biology, 40, 323 332.

Wakeley P.R., Rogers H.Jj., Rozycka M., Greenland A.J., and Hussey P.J., (1998). A maize pectin methylesterase-like gene, Zmc5, specifically expressed in pollen. Plant Molecular Biology, Vol 37, 187-192.

Wu Q., Szakacs-Dobozi M., Hemmat M., and Hrazdina G., (1993). Endopolygalacturonase in apples (*Malus domestica*) and its expression during fruit ripening. Plant Physiol. 102:219-225.

Zamski E., and Peretz I., (1995) Cavity Spot of Carrots - Interactions Between the Host and Pathogen, Related to the Cell-Wall Annals of Applied Biology, Vol 127, 23-32 (TW929).

## Claims

1. A DNA molecule coding for a strawberry polygalacturonase (PG) that comprises a sequence of nucleotides selected from:
a) a sequence of DNA that comprises the sequence of nucleotides identified as SEQ. ID. No.: 1;
b) a sequence of DNA that comprises the sequence of nucleotides identified as SEQ. ID. No.: 5; and
c) a sequence of DNA which is analogous to the sequence defined in a) or in b) which
i) is substantially homologous to the sequence of DNA defined in a) or in b); and/or
ii) codes for a polypeptide that is substantially homologous to the protein coded for by the sequence of DNA defined in a) or in b).

2. A molecule of DNA according to claim 1, in which said DNA is cDNA.

3. A molecule of DNA according to claim 1, in which said DNA is genomic DNA (gDNA).

4. A DNA construct comprising (i) a DNA sequence selected from among a DNA molecule according to any one of claims 1 to 3, and a fragment of at least 8 consecutive nucleotides of said DNA molecule, and (ii) a functional transcription initiation region in plants.

5. A DNA construct according to claim 4, in which the 3' terminal of said DNA sequence is bound to the 3' terminal of said transcription initiation region.

6. A DNA construct according to claim 4, in which the 5' terminal of said DNA sequence is bound to the 3' terminal of said transcription initiation region.

7. A DNA construct according to claim 4, which comprises, in addition, a transcription termination sequence.

8. A recombinant vector that comprises a DNA molecule according to any one of claims 1 to 3, or a DNA construct according to any one of claims 4 to 7.

9. A cell that comprises a DNA molecule according to any one of claims 1 to 3, or a DNA construct according to any one of claims 4 to 6, or a vector according to claim 8.

10. A protein with polygalacturonase activity, obtainable by expression of a DNA molecule according to any one of claims 1 to 3.

11. A protein with polygalacturonase activity that has a sequence of amino acids selected from among:
a) a sequence of amino acids that comprises the sequence of amino acids shown in SEQ. ID. No.: 2,
b) the sequence of amino acids derived from the sequence of nucleotides shown in SEQ. ID. No.: 5, and
c) a sequence of amino acids substantially homologous and functionally equivalent to the sequences of amino acids defined in a) or in b).

12. A protein according to claim 10, which contains a hydrophobic peptide signal at the amino terminal with a potential cleavage site between residues 22 and 23, the cleavage of which yields a mature protein of 40.69 kDa, with a pI of 7.91.

13. A method for the production of a protein with polygalacturonase activity according to any one of claims 10 to 12, which comprises cultivating a cell according to claim 9 under conditions that allow the production of said protein with polygalacturonase activity and recover it from the culture medium.

14. An enzymatic preparation that comprises, at least, a protein according to any one of claims 10 to 12.

15. An enzymatic preparation according to claim 14, which comprises between 0.01% and 100% by weight of a protein according to any one of claims 10 to 12.

16. An enzymatic preparation according to claim 15, which comprises, in addition one or more proteins with different enzymatic activities.

17. An enzymatic preparation according to claim 15, which comprises, in addition, one or more lytic, cellulolytic, mananolytic, chitinolytic or proteolytic enzymes.

18. A method for degrading substrates that contain pectins that comprise the use of a protein according to any one of claims 10 to 12, or an enzymatic preparation according to any one of claims 14 to 17.

19. Use of a protein according to any one of claims 10 to 12, or of an enzymatic preparation according to any one of claims 14 to 17, in the processing of fruits.

20. A method for delaying the softening or for altering the ripening of a soft fruit that comprises introducing into a soft fruit producing plant a DNA construct that has a promoter, functional in said plant, operatively bound to a sub-sequence of DNA of, at least, 8 nucleotides, derived from a DNA molecule according to claims 1 to 3, said DNA sub-sequence being bound to the promoter in the opposite orientation to its expression.

21. A method for delaying the softening or altering the ripening of the strawberry fruit which comprises introducing into a strawberry plant a DNA construct that has a promoter, functional in said plant, operatively bound to a sub-sequence of DNA of, at least, 8 nucleotides, derived from a DNA molecule according to claims 1 to 3, which codes for a strawberry polygalacturonase, said DNA sub-sequence being bound to the promoter in the opposite orientation to its expression.

22. A transgenic cell of a plant that comprises a DNA construct that has a promoter, functional in said plant, operatively bound to a sub-sequence of DNA of, at least, 8 nucleotides, derived from a DNA molecule according to claims 1 to 3, said DNA sub-sequence being bound to the promoter in the opposite orientation to its expression.

23. A transgenic plant that comprises, at least, a transgenic cell according to claim 22.

24. A transgenic plant according to claim 23, in which said plant is a strawberry plant.


LIST OF SEQUENCES

<110> Newbiotechnic, S.A.
<110> University of Cordoba

<120> DNA molecule coding for a polygalacturonase of strawberry
and the uses thereof

<150> P200000579
2000-03-10

<160> 5

<170> PatentIn version 2.0

<210> 1
<211> 599
<212> cDNA
<213> Strawberry

<400> 1
TATCAGTTGG TGATGGTACC AGACAACTAA CTGTAAGTAA GGTAAGTTGT GGACCAGGTC 60
ATGGAATAAG TATCGGAAGT CTCGGAAGAT ACGACAATGA AGATGACGTC AGTGGACTCA 120
ATATTAGAGA TTGTACCCTG AGTAATACAC TGAATGGTGT TAGAATCAAG ACATTTCCTG 180
CTTCTCCTAA AGCTACCACC GCCTCGGATA TTCACTTCGA GAAAATCACA ATGAATAATG 240
TTGCTAACCC AGTCCTCATT GACCAAGAAT ACTGCCCATG GGGTCAGTGT AATAAACAGA 300
TTCCATCAAA AGTCAAGATC AGCAATGTGA GCTTCAAGAA CATTATAGGG ACAACTTCAA 360
CTGCGGAGGC TTTGAAGATT GTATGCGCTA AAGGCTTGCA TTGTGATCAA GTGGTACTGA 420
GTGACATAGA TCTCAAGTTA AGTGGCAAAG GAACTCTTAC ATCTCATTGT GCAAACGTGC 480
AACCCACGAT TACTCGAGTG CCACCGCCTC TTGCTTGTGC TACCAAGCTT GATCATATTG 540
ATTAGCAGCT AGAAATAATA TGGATCTAAA AGACTTAAGT GTCACGTCAA AAAAAAAAA 599

<210> 21
<211> 180
<212> PRT
<213> Strawberry
<400> 2

Ser Val Gly Asp Gly Thr Arg Gln Leu Thr Val Ser Lys Val Ser Cys
1               5                   10                  15

Gly Pro Gly His Gly Ile Ser Ile Gly Ser Leu Gly Arg Tyr Asp Asn
            20                  25                  30

Glu Asp Asp Val Ser Gly Leu Asn Ile Arg Asp Cys Thr Leu Ser Asn
        35                  40                  45

Thr Leu Asn Gly Val Arg Ile Lys Thr Phe Pro Ala Ser Pro Lys Ala
    50                  55                  60

Thr Thr Ala Ser Asp Ile His Phe Glu Lys Ile Thr Met Asn Asn Val
65                  70                  75                  80

Ala Asn Pro Val Leu Ile Asp Gln Glu Tyr Cys Pro Trp Gly Gln Cys
                85                  90                  95

Asn Lys Gln Ile Pro Ser Lys Val Lys Ile Ser Asn Val Ser Phe Lys
            100                 105                 110

Asn Ile Ile Gly Thr Thr Ser Thr Ala Glu Ala Leu Lys Ile Val Cys
        115                 120                 125

Ala Lys Gly Leu His Cys Asp Gln Val Val Leu Ser Asp Ile Asp Leu
    130                 135                 140

Lys Leu Ser Gly Lys Gly Thr Leu Thr Ser His Cys Ala Asn Val Gln
145                 150                 155                 160

Pro Thr Ile Thr Arg Val Pro Pro Pro Leu Ala Cys Ala Thr Lys Leu
                165                 170                 175

Asp His Ile Asp
            180

<210> 3
<211> 31
<212> DNA
<213> Artificial sequence

<223> Oligonucleotide initiator AP1

<400> 3
ACGACTCACT ATAGGGCTTT TTTTTTTTG A 31

<210> 4
<211> 26
<212> DNA
<213> Artificial sequence

<223> Oligonucleotide initiator ARP3

<400> 4
ACAATTTCAC ACAGGAGACC ATTGCA 26

<210> 5
<211> 1755
<212> DNA
<213> Strawberry

<400> 5
TGATTACCAA TTCCAACATT CGCACTTCTT CTACTAGTAG TACTAAAATA GCAAGTAATA 60
TGCATTTGTG GTAAGATGTT TGGTGTTAAC CTTTCCTAAC CAGACTATAA ATGACCTCAA 120
CACTATAGTG GAGTTTCATC GATCATCATT CTAAACGAAA AACTTGAAGT GAAAGCATCA 180
AGATGGGAGG ACACAATCTA GTTTTGATTA TCTTTTCGGT ATTTTTGTTA TCTTCCTCAG 240
CATATGCAAG TAGAGTCGCA CAGTTTTTAG CTCCTGGTGA CTTTGATGTG ACAAGTCCGA 300
AATATGGTGG GAAGCCTAAT ACTGATATTA GTCAGGTAAA CTATACATAT TATAATCGTA 360
CAAATACTTC AAAATAGAAT GGTTGATTTC ATTTTTCTTT TTCTTTGTAC ATAGCCTTTG 420
GCAAATGCTT GGAAAGATGC TTGTGCAGCA ACAACGCCAA GTAGAGTTAT TGTTCCAAAA 480
GGGACATTTC AACTAAAAGG AGCAGTTTTC AAAGGTCCTT GTAAGGCTCC TATTACGGTT 540
CAGGTTGATG GCATACTGCA GGCGCCACCA ATTGAAGCCC AATTAGCAAA TAAAGAATTT 600
TGGGTCCAGT TTTTAGAGGT TGAGAGGCTC ACTGTATCAG GTACTGGAAC ATTTGATGGT 660

CAAGGACAAA ATTCTTGGAA AGACAACGAG TGCAATAAAA ATCCAAACTG TGGAGGTCTA 720
GCGATTGTAA GCTAATATAG CCCTCTTCCC TGTTTATTTA TTTATTTATT TTTTGATGTA 780
TTGCATTTCG TGCTAATTTG TTTCAGGTAA CCCTGATTTA CATTTTCAAG ATATATTGAT 840
ATATATATAT ATGTCGTGCT GCTTTCTCAT TATGTGCAGA ATGTGAGATT CGACAGAGTG 900
AAAAATTCGT TAGTAAGGGA TGTGACATCA CTTAACAGCA AAAATTTCCA CTTCAATATT 960
TTAGGGTGTG AACATCTTAC ATTCCAACAT GTCATCGTCA AAGCACCGGG AGATAGCCCT 1020
AACACAGATG GAATACATAT GGGGCCCTCA ACCAGGATTA ACATTACGGA ACAGAACATC 1080
CGAACCGGAG ACGATTGCAT ATCAGTTGGT GATGGTACCA GACAACTAAC TGTAAGTAAG 1140
GTAAGTTGTG GACCAGGTCA TGGAATAAGT ATCGGAAGTC TCGGAAGATA CGACAATGAA 1200
GATGACGTCA GTGGACTCAA TATTAGAGAT TGTACCCTGA GTAATACACT GAATGGTGTT 1260
AGAATCAAGA CATTTCCTGC TTCTCCTAAA GCTACCACCG CCTCGGATAT TCACTTCGAG 1320
AAAATCACAA TGAATAATGT TGCTAACCCA GTCCTCATTG ACCAAGAATA CTGCCCATGG 1380
GGTCAGTGTA ATAAACAGGT AATCATCCAT GCATCTTAAT TACTAACCTA CATAATACTT 1440
CTTTTTCGCG TCTTGGTTAC CGATGTACCT CATGATATAT GTAGTCTATC AGGTTTCTTA 1500
ATTAAATTCC ATTATCCATG ATCTTATTAA TTATACACAA ATCTAATTAG TGATGTTTCT 1560
TGCTCGAAGA TTCCATCAAA AGTCAAGATC AGCAATGTGA GCTTCAAGAA CATTATAGGG 1620
ACAACTTCAA CTGCGGAGGG TTTGAAGATT GTATGCGCTA AAGGCTTGCA TTGTGATCAA 1680
GTGGTACTGA GTGACATAGA TCGAGTGGAC GCCCTATAGT GAGTCGTATT AGAGCTCGGG 1740
GCCGCGAGCT CTAGA 1755